Europäisches Patentamt

European Patent Office

Office européen des brevets

(11) Publication number: **0 259 858 B1**

## EUROPEAN PATENT SPECIFICATION

(45) Date of publication of patent specification: **31.03.93**

(51) Int. Cl.5: **C12N 15/00**, C12P 13/10, C12N 9/00, C12N 1/20, //(C12N1/20,C12R1:15,1:13)

(21) Application number: **87113188.4**

(22) Date of filing: **09.09.87**

(54) **Process for producing amino acids.**

(30) Priority: **10.09.86 JP 213572/86**

(43) Date of publication of application:
**16.03.88 Bulletin 88/11**

(45) Publication of the grant of the patent:
**31.03.93 Bulletin 93/13**

(84) Designated Contracting States:
**DE FR GB**

(56) References cited:
**EP-A- 0 077 548**
**EP-A- 0 136 359**
**FR-A- 2 484 448**

**PATENT ABSTRACTS OF JAPAN, vol. 6, no. 63 (C-99)(941), 22 April 1982**

**PATENT ABSTRACTS OF JAPAN, vol. 9, no. 196, (C-297)(1919), 13 August 1985**

**ENZYME NOMENCLATURE 1978, Academic Press, New York, NY (US); pp. 9, 66**

(73) Proprietor: **KYOWA HAKKO KOGYO CO., LTD.**
**6-1, Ohtemachi 1-chome**
**Chiyoda-ku Tokyo-to(JP)**

(72) Inventor: **Katsumata, Ryoichi**
**CI-Heights H-801, 1380, Yamazaki-machi**
**Machida-shi Tokyo(JP)**
Inventor: **Yokoi, Haruhiko**
**Popuragaoka Coopu 21-205 2-32-4,**
**Narusedai**
**Machida-shi Tokyo(JP)**

(74) Representative: **Vossius & Partner**
**Siebertstrasse 4 P.O. Box 86 07 67**
**W-8000 München 86 (DE)**

EP 0 259 858 B1

**Description**

This invention relates to a process for producing amino acids comprising culturing a microorganism belonging to the genus Corynebacterium or Brevibacterium carrying a recombinant DNA.

In the production of L-arginine by fermentation using coryneform glutamate-producing bacteria of the genus Corynebacterium or Brevibacterium, mutants having an ability to produce L-arginine and derived from wild type strains of these bacteria have been employed.

As the L-arginine-producing mutants, mutants resistant to amino acid analogues or nucleic acid analogues or mutants obtained by imparting auxotrophy for nucleic acid bases to these analogue-resistant strains, etc. are known as described in Agric. Biol. Chem., 36, 1675 (1972), Japanese Published Examined Patent Application No. 37235/1979, Japanese Published Unexamined Patent Application No. 150381/1982, etc. There is also known a method of producing L-arginine using strains constructed by recombinant DNA technology. For example, there is known a method or producing L-arginine by culturing a bacterium belonging to the genus Corynebacterium or Bevibacterium and carrying a recombinant plasmid DNA (PEargl) containing a DNA fragment isolated from Escherichia coli K12 and bearing genetic information responsible for biosynthesis of arginine, inter alia, genes coding for N-acetylglutamate kinase (hereafter referred to as AGK), N-acetyl-γ-glutamylphosphate reductase (hereafter referred to as AGPR), acetylornithine deacetylase (hereafter referred to as AOD) and argininosuccinase (hereafter referred to as AS) (Japanese Published Unexamined Patent Application No. 66989/1985; EP-A-0136359).

Further, with respect to the method of producing L-ornithine or L-citrulline by fermentation using coryneform glutamate-producing bacteria of the genus Corynebacterium or Brevibacterium, a method using nutrient-requiring mutants derived from wild type strains of these bacteria is known. As the L-ornithine-producing mutants, there are known mutants to which citrulline- or arginine-requirement has been imparted, etc., as described in J. Gen. Appl. Microbiol., 4, 272 (1958). As the L-citrulline-producing mutants, there are known mutants to which L-arginine-requirement has been imparted, etc., as described in Amino Acid Nucleic Acid, 14, 6 (1966), etc.

With the recent increase in the demand for L-arginine, L-ornithine and L-citrulline, improved processes for the industrial production thereof are desired.

The present inventors have made extensive studies in order to obtain bacteria belonging to the genus Corynebacterium or Brevibacterium and having an improved productivity of L-arginine, L-citrulline or L-ornithine by recombinant DNA technology. As a result, it has been found that when bacteria belonging to the genus Corynebacterium or Brevibacterium carrying a novel recombinant plasmid (pEarg2) are used, productivity of L-arginine can be enhanced as compared to the production method using pEarg1-carrying bacteria. pEarg2 differs from a recombinant plasmid pEarg1 containing the genes derived from Escherichia coli K12 and coding for AGK, AGPR, AOD and AS in that pEarg2 is deficient of the gene coding for AOD. It has also been found that pEarg2-carrying bacteria have an enhanced productivity of L-ornithine or L-citrulline as compared to pEarg2-free bacteria, and further that when bacteria belonging to the genus Corynebacterium or Brevibacterium carrying a recombinant plasmid DNA (pEarg4) constructed by incorporating a DNA fragment derived from Escherichia coli K12 and containing the gene coding for argininosuccinate synthetase (hereafter referred to as ASS) into pEarg2 are used, such bacteria have L-arginine-productivity higher than that of the pEarg2-carrying bacteria. The present inventors have thus accomplished the present invention.

The present invention relates to a process for producing L-arginine, L-citrulline or L-ornithine which comprises culturing a microorganism belonging to the genus Corynebacterium or Brevibacterium carrying a recombinant DNA of a DNA fragment bearing genetic information responsible for synthesis of AGK, AGPR and AS which are isolated from strains belonging to the genus Escherichia, and a vector DNA in a medium until recoverable amounts of L-arginine, L-citrulline or L-ornithine are accumulated in the culture broth and recovering said amino acid therefrom. Further, the present invention relates to a process for producing L-arginine which comprises culturing a microorganism belonging to the genus Corynebacterium or Brevibacterium carrying a recombinant DNA of DNA fragments bearing genetic information responsible for synthesis of AGK, AGPR, AS and ASS which are isolated from strains belonging to the genus Escherichia, and a vector DNA in a medium until recoverable amounts of L-arginine are accumulated in the culture broth and recovering L-arginine therefrom. Accordingly, the present invention relates to the field of bio-industry and more particularly to the field of production of L-arginine, L-citrulline or L-ornithine useful for drugs and foodstuffs.

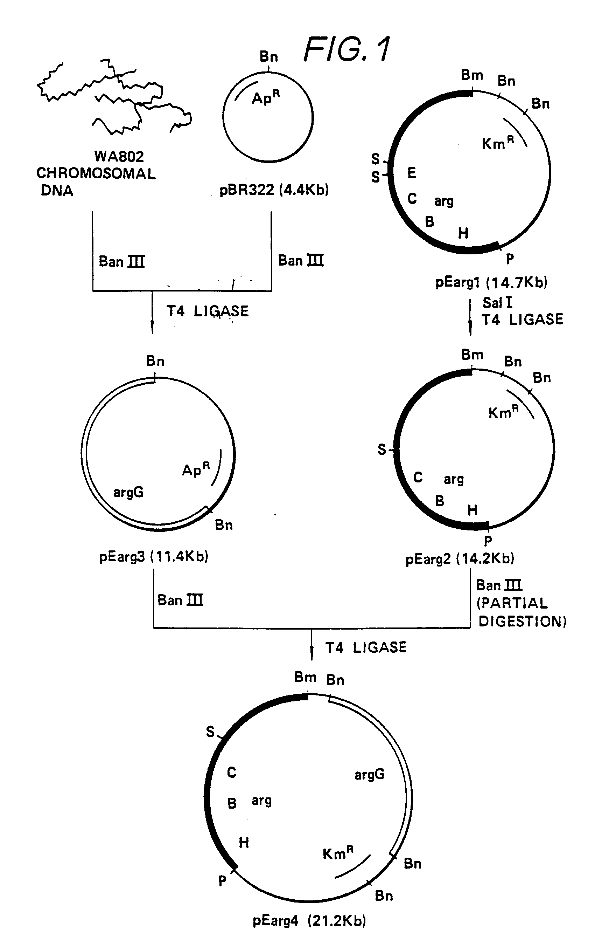

Figure 1 shows a cleavage map of pEarg2, pEarg3 and pEarg4 with restriction enzymes BamHI, BanIII, PstI and SalI and the steps for constructing thereof. In figure, Bm, Bn, P and S indicate respectively BamHI, BanIII, PstI and SalI. The size of the plasmid is shown by kilobase (Kb). Bold solid portions and white-on-

2

black bold portions of pEarg2, pEarg3 and pEarg4 indicate a DNA fragment containing a series of the arginine biosynthesis genes (argB, argC and argH genes) derived from pEarg1 and a DNA fragment containing argG gene cloned from chromosomal DNA of the WA802 strain, respectively.

As the microorganisms belonging to the genus Corynebacterium or Brevibacterium to be usable as the host microorganism of the present invention, all microorganisms which are known to be the so-called coryneform glutamate-producing bacteria can be employed. The following strains are exemplary of those suitable for transformation:

Corynebacterium glutamicum ATCC 31833
Corynebacterium acetoacidophilum ATCC 13870
Corynebacterium herculis ATCC 13868
Corynebacterium lilium ATCC 15990
Brevibacterium divaricatum ATCC 14020
Brevibacterium flavum ATCC 14067
Brevibacterium immariophilum ATCC 14068
Brevibacterium lactofermentum ATCC 13869
Brevibacterium thiogenitalis ATCC 19240

In the case that L-arginine is produced, bacteria having no L-arginine productivity are used as the host microorganism but L-arginine producing bacteria can preferably be used. As the bacteria having L-arginine productivity, known strains such as amino acid analogue-resistant mutants, etc. can be employed. In the case of producing L-ornithine or L-citrulline, arginine-requiring strains having L-ornithine or L-citrulline productivity are used as the host microorganism.

As a supply source of the genes coding for AGK, AGPR, AS and ASS, any microorganism can be used as far as it is a microorganism belonging to the genus Escherichia and has the activity of AGK, AGPR, AS and ASS. As a specific preferred example, Escherichia coli K12 strain is mentioned. In the Escherichia coli K12 strain, AGK, AGPR, AS and ASS are encoded on argB, argC, argH and argG genes, respectively [Genetics, 51, 167 (1965)].

The chromosomal DNA used as a supply source of these genes can be isolated from microorganisms having AGK, AGPR, AS and ASS activity in a conventional manner [Biochim. Biophys. Acta, 72, 619 (1963)] which comprises treating the cultured cells of the microorganism with lysozyme and a surfactant for bacteriolysis, removing proteins and then precipitating the DNA with ethanol.

The vector to be used in the present invention is restricted only to the extent that it is autonomously replicable in bacteria belonging to the genus Corynebacterium or Brevibacterium. Particularly suitable plasmids are: pCG1 (Japanese Published Unexamined Patent Application No. 134500/1982), pCG2 (Japanese Published Unexamined Patent Application No. 35197/1983), pCG4 and pCG11 (Japanese Published Unexamined Patent Application No. 183799/1982), pCE51, pCE52 and pCE53 [Mol. Gen. Genet., 196, 175 (1984)], pCE54 and pCB101 (Japanese Published Unexamined Patent Application No. 105999/1983) or plasmids derived from these vector plasmids. The plasmid can be isolated and purified from the culture cells of the strain carrying the plasmid in such a manner as disclosed in Japanese Published Unexamined Patent Applications Nos. 134500/1982 and 186489/1982.

The recombinant DNA of the donor DNA bearing the genes coding for AGK, AGPR, AS and ASS and the vector DNA can be obtained by digesting both DNAs with restriction enzymes in vitro, carrying out a ligation reaction with DNA ligase, transforming AGK-, AGPR-, AS- or ASS-deficient mutants belonging to the genus Corynebacterium or Brevibacterium with the ligation reaction mixture and selecting a transformant wherein the deficient character is complemented. The procedure is carried out according to the method described in Japanese Published Unexamined Patent Applications Nos. 186492/1982 and 186489/1982.

Instead of directly obtaining the desired recombinant DNA from a transformant of bacteria belonging to the genus Corynebacterium or Brevibacterium, there may also be used the host-vector system of Escherichia coli wherein the gene recombinant DNA technology has been established. Namely, the desired genes can be cloned by digesting, with restriction enzymes, the donor DNA bearing the genes coding for AGK, AGPR, AS and ASS and the vector DNA of Escherichia coli in vitro, ligating both DNAs with DNA ligase, transforming AGK-, AGPR-, AS- or ASS-deficient mutants of Escherichia coli with the ligation reaction mixture and selecting a transformant wherein the deficient character is complemented. The desired recombinant DNA is obtained by digesting the thus cloned DNA and the vector DNA of bacteria belonging to the genus Corynebacterium or Brevibacterium with restriction enzymes in vitro, ligating both DNAs again with DNA ligase, transforming the aforesaid AGK-, AGPR-, AS- or ASS-deficient mutants of Escherichia coli with the ligation reaction mixture and selecting a transformant having a selection marker of the vector DNA of bacteria belonging to the genus Corynebacterium or Brevibacterium wherein the deficient character is complemented. The desired recombinant DNA can also be obtained by transforming mutants of bacteria belonging to the genus Corynebacterium or Brevibacterium that are deficient of AGK, AGPR, AS or ASS with the ligation reaction mixture and selecting a transformant wherein the deficient character is complemented, instead of transforming Escherichia coli with the ligation reaction mixture upon the selection of the recombinant of the DNA cloned in Escherichia coli and the vector DNA of bacteria belonging to the genus Corynebacterium or Brevibacterium.

Further, the recombinant DNA of the donor DNA bearing the genes coding for AGK, AGPR and AS and the vector DNA can be obtained by the same method as described above.

Instead of using the chromosomal DNA as a supply source of the genes coding for AGK, AGPR and AS, the DNA fragment containing the genes coding for AGK, AGPR and AS which have already been cloned on pEarg1 can also be used. As mentioned above, pEarg1 contains a gene coding for AOD (argE) in addition to the genes coding for AGK, AGPR and AS (argB, argC and argH). pEarg1 is disclosed in Japanese Published Unexamined Patent Application No. 66989/1985, and is a recombinant of plasmid pLC20-10 in the gene bank of Escherichia coli K12 strain [Cell, 9, 91 (1976)] and plasmid pCE53 [Mol. Gen. Genet., 196, 175 (1984)]. A plasmid containing only argB, argC and argH genes can be prepared by deleting part of a DNA fragment corresponding to the argE gene as described in, for example, Example 1.

Cloning of the argG gene can be carried out by the method described above, using chromosomal DNA of a strain derived from Escherichia coli K12 having the ASS activity, for example, WA802 strain (FERM BP-718).

Further, a recombinant DNA containing all of argB, argC, argH and argG genes can be obtained by ligating, for example, the DNA fragment containing argG gene and the plasmid containing argB, argC and argH genes described above.

The recombinant DNA containing all of argB, argC and argH genes, or the recombinant DNA containing all of argB, argC, argH and argG genes obtained above can be introduced into bacteria belonging to the genus Corynebacterium or Brevibacterium by the method using a protoplast shown in Japanese Published Unexamined Patent Applications Nos. 186492/1982 and 186489/1982.

Production of L-arginine, L-citrulline or L-ornithine using these recombinant DNA-carrying strains can be carried out in a conventional manner used for the production of these amino acids by fermentation. That is, the transformant is cultured in an ordinary medium containing carbon sources, nitrogen sources, inorganic compounds, amino acids, vitamins, etc. under aerobic conditions while adjusting temperature, pH, etc., whereby L-arginine, L-citrulline or L-ornithine is accumulated in the culture broth and such amino acids are recovered from the culture broth.

As the carbon source, there can be used carbohydrates such as glucose, glycerol, fructose, sucrose, maltose, mannose, starch, starch hydrolysate, molasses, etc.; polyalcohols; various organic acids such as pyruvic acid, fumaric acid, lactic acid, acetic acid, etc. Further, depending upon assimilation of bacteria, there may also be used hydrocarbons, alcohols, etc. In particular, cane molasses is preferably used.

As the nitrogen source, there can be used ammonia or various inorganic and organic ammonium salts such as ammonium chloride, ammonium sulfate, ammonium carbonate, ammonium acetate, etc.; urea and other nitrogen-containing compounds as well as nitrogen-containing organic compounds such as peptone, NZ-amine, meat extract, yeast extract, corn steep liquor, casein hydrolysate, fish meal or its digested products, defatted soybean lees or digested products thereof, chrysalis hydrolysate, etc.

Further, as the inorganic compounds, there can be used potassium dihydrogenphosphate, dipotassium hydrogenphosphate, ammonium sulfate, ammonium chloride, magnesium sulfate, sodium chloride, ferrous sulfate, manganese sulfate, calcium carbonate, etc. If vitamins, amino acids, etc. required for the growth of the microorganism are supplied by other medium components described above, it is not necessary to add these specific nutrients separately to the medium.

Culturing is carried out under aerobic conditions by shaking culture, aeration-stirring culture, etc. The preferred culturing temperature is 20 to 40°C. It is desired that the pH of medium during culturing be maintained around the neutrality. After culturing for 1 to 5 days under these conditions, recoverable quantities of L-arginine, L-citrulline or L-ornithine are accumulated in the culture broth.

After completion of the culturing, the cells are removed from the culture broth, and L-arginine, L-citrulline or L-ornithine is recovered from the supernatant by known methods such as a treatment with activated carbon, a treatment with ion exchange resins, etc.

With bacteria belonging to the genus Corynebacterium or Brevibacterium carrying the recombinant DNA containing all of argB, argC and argH genes as described above, L-arginine, L-citrulline or L-ornithine can be produced in a higher yield than with strains carrying no recombinant DNA; further with bacteria belonging to the genus Corynebacterium or Brevibacterium carrying the recombinant DNA containing all of argB, argC, argH and argG genes, L-arginine can be produced in a higher yield than with strains carrying no recombinant DNA. Specifically, Corynebacterium glutamicum K62 having arginine productivity (FERM BP-1112) and Corynebacterium glutamicum K63 having arginine productivity (FERM BP-1113) carry such recombinant DNAs and have been deposited in Fermentation Research Institute, Agency of Industrial Science and Technology, Japan as of July 24, 1986, together with citrulline-producing strain, Corynebacterium glutamicum K61 (FERM BP-1111).

Hereafter the present invention will be described by referring to examples below.

Example 1

(1) Preparation of arginine-requiring mutant of Escherichia coli:

In order to carry out cloning of an arginine biosynthetic gene and to ascertain the cloned gene, an Escherichia coli strain deficient of the arginine biosynthetic gene was obtained as follows.

Escherichia coli WA802 having methionine-requirement (FERM BP-718) derived from Escherichia coli K12 was subjected to ordinary mutation treatment with 400 γ/mℓ N-methyl-N'-nitro-N-nitrosoguanidine (NTG). After concentration in accordance with the method for concentration of a nutrient-requiring strain using penicillin [Experiment in Molecular Genetics, p. 230, Cold Spring Harbour Laboratory (1972)], the arginine-requiring strains were selected. Identification of the mutation genes of the arginine-requiring strains obtained was carried out by determining the activity of each arginine biosynthetic enzyme.

The enzyme assay was carried out as described in J. Gen. Microbiol., 69, 365 (1971) with respect to AGK, AOD and AS; in Biochim. Biophys. Acta, 108, 306 (1965) with respect to AGPR; and in J. Biochem., 85, 1309 (1979) with respect to ASS. As a result, EA-21, EA-35, EA-4, EA-51 and EA-77 were obtained as AGK-deficient mutant (argB), AGPR-deficient mutant (argC), AOD-deficient mutant (argE), ASS-deficient mutant (argG) and AS-deficient mutant (argH), respectively.

(2) Production of plasmid DNA containing argB, argC and argH genes and no argG gene of Escherichia coli:

Recombinant DNA pEarg1 containing clusters comprising argB, argC, argH and argE genes of Escherichia coli can be obtained by ligating pLC20-10 and pCE53, as shown in Japanese Published Unexamined Patent Application No. 66989/1985. It is known that pLC20-10 contains the gene group described above [Cell, 9, 91 (1976)]. pLC20-10 is obtained from the gene bank of Escherichia coli K12 strain. pCE53 is a plasmid described in Mol. Gen. Genet., 196, 175 (1984), having kanamycin-resistant gene as a selection marker and being autonomously replicable in both of Escherichia coli and coryneform glutamate-producing bacteria belonging to the genus Corynebacterium or Brevibacterium. pEarg1 has cleavage sites with various restriction enzymes as shown in Figure 1, in which the two cleavage sites with SalI are both present in the argE gene [Gene, 5, 207 (1979)].

Plasmid DNA containing all of argB, argC and argH genes and no argE gene was obtained from pEarg1 as follows. pEarg1 plasmid DNA was isolated from the culture cells of Escherichia coli WA802 derived from Escherichia coli K12 and carrying pEarg1 according to the method of Ann, et al. [J. Bacteriol., 140, 400 (1979)]. Five units of SalI (product of Takara Shuzo Co., Ltd., 8 units/μℓ) were added to 100 μℓ of a reaction buffer solution for restriction enzyme SalI [10 mM tris(hydroxymethyl)aminomethane (hereafter referred to as Tris), 150 mM NaCl and 7 mM MgCl₂, pH 8.0] containing 2 μg of pEarg1 plasmid DNA followed by reaction at 37°C for an hour. After heating at 65°C for an hour, 12 μℓ of T4 ligase reaction buffer solution at a 10-fold concentration (660 mM Tris, 66 mM MgCl₂ and 100 mM dithiothreitol, pH 7.6), 3 μℓ of ATP (100 mM) and 1 μℓ of T4 ligase (product of Takara Shuzo Co., Ltd., 350 units/μℓ) were added to the said digested product followed by reaction at 4°C for 24 hours. This ligase reaction mixture was used for transformation. As a recipient, AOD-deficient Escherichia coli EA-4 (requiring arginine and methionine) derived from Escherichia coli K12 obtained in (1) was used. Competent cells of EA-4 were prepared by the method of Dagert, et al. [Gene, 6, 23 (1979)].

Namely, EA-4 strain was inoculated in 50 mℓ of L medium (which is a medium containing 10 g of Bactotrypton, 5 g of yeast extract, 1 g of glucose and 5 g of NaCl in 1 liter of water, adjusted to pH 7.2) and cultured at 37°C until the absorbance (OD) at 660 nm, determined with a Tokyo Koden colorimeter, reached 0.5 (absorbance was determined at 660 nm, unless otherwise indicated). The culture broth was cooled on ice for 10 minutes and then centrifuged. The cells were resuspended in 20 mℓ of 0.1 M CaCl₂ and the suspension was allowed to stand for 20 minutes at 0°C. The cells were again centrifuged and suspended in 0.5 mℓ of 0.1 M CaCl₂ and the suspension was allowed to stand for 18 hours at 0°C. To 150 μℓ of the cell suspension treated with CaCl₂ were added 50 μℓ of the ligase reaction mixture described above. The mixture was allowed to stand at 0°C for 10 minutes and then heated at 37°C for 5 minutes. Subsequently, 2 mℓ of L medium was added thereto followed by shaking the culture at 37°C for 2 hours.

After washing twice with physiological saline solution by centrifugation, the washed cells were spread on L agar medium (medium containing 16 g of agar in 1 liter of L medium) supplemented with 25 $\mu$g/m$\ell$ kanamycin, and cultured at 37°C for one day. The appearing transformants were spread on Davis minimal agar medium [containing 2 g of glucose, 1 g of $(NH_4)_2SO_4$, 7 g of $K_2HPO_4$, 2 g of $KH_2PO_4$, 0.1 g or $MgSO_4 \cdot 7H_2O$, 0.5 g of trisodium citrate, 4 mg of thiamine hydrochloride and 16 g of agar in 1 liter of water, adjusted to pH 7.2] supplemented with 30 $\mu$g/m$\ell$ methionine, and on Davis minimal agar medium supplemented with 30 $\mu$g/m$\ell$ each of methionine and arginine to examine arginine auxotrophy. A plasmid DNA was isolated from the culture cells of the transformants showing arginine auxotrophy in a manner similar to the isolation of pEarg1 described above. This plasmid DNA was analyzed by digestion with restriction enzymes and agarose gel electrophoresis. As the result, the plasmid was shown to have a structure wherein a DNA fragment of 0.5 Kb located between two cleavage sites with SalI of pEarg1 was deleted as shown in Figure 1. This plasmid was named pEarg2.

The EA-4 strain was retransformed with this plasmid DNA in a manner as described above. As the result, all of the thus obtained kanamycin-resistant transformants had arginine-requirement. However, transformation of AGK-deficient EA-21, AGPR-deficient EA-35 and AS-deficient EA-77 (all of which have arginine- and methionine-requirement) derived from Escherichia coli K12 with pEarg2 gave kanamycin-resistant transformants requiring no arginine. From this fact, it was noted that pEarg2 was a plasmid containing all of argB, argC and argH genes and no argE gene (cf. Figure 1).

(3) Production of L-arginine, L-citrulline and L-ornithine by pEarg2-carrying strain

Protoplasts of Corynebacterium glutamicum ATCC 31833, Corynebacterium herculis ATCC 13868, Brevibacterium flavum ATCC 14067, L-ornithine-producing strain Corynebacterium glutamicum ATCC 13232 and citrulline-producing strain Corynebacterium glutamicum K61 (FERM BP-1111) isolated as arginine-requiring mutant from Corynebacterium glutamicum ATCC 13032 were transformed with pEarg2. Corynebacterium glutamicum ATCC 31833, Corynebacterium herculis ATCC 13868, Brevibacterium flavum ATCC 14067, Corynebacterium glutamicum ATCC 13232 and Corynebacterium glutamicum K61 (FERM BP-1111) were cultured by shaking at 30°C overnight in NB medium (medium containing 20 g of bouillon powders and 5 g of yeast extract, adjusted to pH,7.2) and 0.1 m$\ell$ of each of the seed cultures was inoculated in an L-test tube containing 10 m$\ell$ of SSM medium [medium containing 10 g of glucose, 4 g of $NH_4Cl$, 2 g of urea, 1 g of yeast extract, 1 g of $KH_2PO_4$, 3 g of $K_2HPO_4$, 0.4 g of $MgCl_2 \cdot 6H_2O$, 10 mg of $FeSO_4 \cdot 7H_2O$, 0.2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 $\mu$g of biotin and 1 mg of thiamine hydrochloride in 1 liter of distilled water, adjusted to pH 7.2] or 10 m$\ell$ of SSM medium supplemented with 200 $\mu$g/m$\ell$ arginine, followed by shaking culture at 30°C. At the time when OD reached 0.15, penicillin G was added to a final concentration of 0.5 units/m$\ell$. Culturing was continued and the cells were collected when OD reached about 0.6. The cells were suspended in a concentration of about $10^9$ cells/m$\ell$ in 2.5 m$\ell$ of RCGP medium [medium containing 5 g of glucose, 5 g of casamino acid, 2.5 g of yeast extract, 3.5 g of $K_2HPO_4$, 1.5 g of $KH_2PO_4$, 0.41 g of $MgCl_2 \cdot 6H_2O$, 10 mg of $FeSO_4 \cdot 7H_2O$, 2 mg of $MnSO_4 \cdot 4-6H_2O$, 0.9 mg of $ZnSO_4 \cdot 7H_2O$, 0.04 mg of $(NH_4)_6Mo_7O_{24} \cdot 4H_2O$, 30 $\mu$g of biotin, 2 mg of thiamine hydrochloride, 135 g of disodium succinate and 30 g of polyvinyl pyrrolidone having a molecular weight of 10,000 in 1 liter of water] supplemented with 1 mg/m$\ell$ lysozyme (pH 7.6). The suspension was transferred to the L-test tube and cultured by shaking gently at 30°C for 15 hours to induce protoplast formation. Then, 0.5 m$\ell$ of the protoplast suspension was taken into a small test tube, centrifuged at 2,500 x g for 5 minutes and suspended in 1 m$\ell$ of TSMC buffer solution [10 mM $MgCl_2$, 30 mM $CaCl_2$, 50 mM Tris and 400 mM sucrose; pH 7.5]. The suspension was washed by centrifugation and the washed cell were resuspended in 0.1 m$\ell$ of TSMC buffer solution. To the cell suspension were added 100 u$\ell$ of a 1:1 mixture of TSMC buffer solution at a 2-fold concentration and pEarg2 plasmid DNA solution was added to the cell suspension. Then, 1.0 m$\ell$ of a solution of 20% polyethylene glycol (PEG) 6,000 (product of Nakarai Chemical Co., Ltd.) in TSMC buffer solution was added thereto. After 3 minutes, the mixture was centrifuged at 2,500 x g for 5 minutes and the supernatant was removed. The precipitated protoplasts were suspended in 1 m$\ell$ of RCGP medium (pH 7.4) and the suspension was gently shaken at 30°C for 2 hours. Then, 0.3 m$\ell$ of the protoplast suspension was spread onto RCGP medium (pH 7.4) containing 1.6% agar and 400 $\mu$g/m$\ell$ kanamycin followed by culturing at 30°C for 8 days.

The appearing kanamycin-resistant transformant was cultured by shaking in 400 m$\ell$ of SSM medium and, penicillin G was added thereto to a final concentration of 0.5 units/m$\ell$ when OD reached 0.15. The culturing was continued until OD reached 0.65. The cells were collected from the culture broth. After washing with TES buffer solution (0.03 M Tris, 0.005 M EDTA and 0.05 M NaCl; pH 8.0), the cells were suspended in 10 mℓ of a lysozyme solution (25% sucrose, 0.1 M NaCl, 0.05 M Tris and 0.8 mg/mℓ lysozyme; pH 8.0) followed by reaction at 37°C for 4 hours. To the reaction mixture were added, in order, 2.4 mℓ of 5 M NaCl, 0.6 mℓ of 0.5 M EDTA (pH 8.0) and 4.4 mℓ of a solution consisting of 4% sodium laurylsulfate and 0.7 M NaCl. After the mixture was gently mixed, the mixture was placed on ice water for 15 hours. The whole amount of the bacteriolytic product was transferred to a centrifuge tube and centrifuged at 69,400 x g for 60 minutes at 4°C to recover the supernatant. PEG 6,000 (product of Nakarai Chemical Co., Ltd.) in an amount corresponding to 10% by weight was added to the supernatant, the mixture was dissolved by gently stirring, and then placed on ice. After 10 hours, the mixture was centrifuged at 1,500 x g for 10 minutes to recover pellets. Then, 5 mℓ of TES buffer solution were added to gradually dissolve the pellets. Then, 2.0 mℓ of 1.5 mg/mℓ ethidium bromide were added to the solution. Cesium chloride was added to adjust the density of the solution to 1.580. The solution was subjected to ultra-centrifugation at 105,000 x g at 18°C for 48 hours. By this density gradient centrifugation, the cyclic DNA closed by covalent bond was found to be a band having a high density at the lower position of the centrifuge tube detected under ultraviolet irradiation. The band was withdrawn from the side of the centrifuge tube through a syringe, whereby plasmid was separated. Then, the separated solution was treated 5 times with an equal volume of an isopropyl alcohol solution [90% by volume isopropyl alcohol and 10% by volume TES buffer solution (containing a saturation amount of cesium chloride in this mixture)] to remove ethidium bromide by extraction; thereafter the mixture was dialyzed against TES buffer solution to obtain a final plasmid DNA preparation.

The plasmid was analyzed by digestion with various restriction enzymes and agarose gel electrophoresis. As the result, it was determined that the plasmid had the same structure of pEarg2 characterized by cleavage patterns with various restriction enzymes.

Thus, the transformants, Corynebacterium glutamicum ATCC 31833/pEarg2, Corynebacterium herculis ATCC 13868/pEarg2, Brevibacterium flavum ATCC 14067/pEarg2, Corynebacterium glutamicum ATCC 13232/pEarg2 and Corynebacterium glutamicum K61/pEarg2 were obtained. Among them, Corynebacterium glutamicum ATCC 31833/pEarg2 has been deposited in Fermentation Research Institute, Agency of Industrial Science and Technology, Japan under Corynebacterium glutamicum K62 (FERM BP-1112).

Further, pEarg1 was introduced into Corynebacterium glutamicum ATCC 31833, Corynebacterium herculis ATCC 13868, Brevibacterium flavum ATCC 14067, Corynebacterium glutamicum ATCC 13232 and Corynebacterium glutamicum K61 in a similar manner to obtain the respective transformants.

With respect to the thus obtained pEarg1 or pEarg2-carrying transformants, production test of L-arginine, L-citrulline or L-ornithine was carried out as follows.

0.5 mℓ of the seed culture obtained by shaking the culture at 30°C for 16 hours in NB medium were inoculated in 5 mℓ of production medium [medium containing 80 g of cane molasses (calculated as glucose), 40 g of $(NH_4)_2SO_4$, 0.5 g of $KH_2PO_4$, 0.5 g of $K_2HPO_4$ and 20 g of $CaCO_3$ in 1 liter of water, adjusted to pH 7.0] charged in a test tube followed by shaking the culture at 30°C for 72 hours. In the production test of L-citrulline or L-ornithine, 100 μg/mℓ L-arginine were supplemented to 5 mℓ of the production medium. After culturing, the culture filtrate was subjected to paper chromatography and after color formation with ninhydrin, colorimetry was carried out to determine the amount of L-arginine, L-citrulline and L-ornithine produced.

EP 0 259 858 B1

The results are shown in Tables 1, 2 and 3.

## Table 1

| Strain | Amount of L-Arginine Produced (mg/mℓ) |
|---|---|
| Corynebacterium glutamicum ATCC 31833 | 0 |
| Corynebacterium glutamicum ATCC 31833/pEarg1 | 1.6 |
| Corynebacterium glutamicum ATCC 31833/pEarg2 (K62 FERM BP-1112) | 1.9 |
| Corynebacterium herculis ATCC 13868 | 0 |
| Corynebacterium herculis ATCC 13868/pEarg1 | 1.8 |
| Corynebacterium herculis ATCC 13868/pEarg2 | 2.1 |
| Brevibacterium flavum ATCC 14067 | 0 |
| Brevibacterium flavum ATCC 14067/pEarg1 | 1.0 |
| Brevibacterium flavum ATCC 14067/pEarg2 | 1.4 |

## Table 2

| Strain | Amount of L-Citrulline Produced (mg/mℓ) |
|---|---|
| Corynebacterium glutamicum K61 (FERM BP-1111) | 1.0 |
| Corynebacterium glutamicum K61/pEarg1 | 1.8 |
| Corynebacterium glutamicum K61/pEarg2 | 6.0 |

8

Table 3

| Strain | Amount of L-Ornithine Produced (mg/mℓ) |
|---|---|
| Corynebacterium glutamicum ATCC 13232 | 18.9 |
| Corynebacterium glutamicum ATCC 13232/pEarg1 | 11.3 |
| Corynebacterium glutamicum ATCC 13232/pEarg2 | 21.0 |

From the foregoing results, it is apparent that the productivity of arginine, citrulline or ornithine can be improved with the strains carrying plasmid pEarg2 obtained by removing argE gene from pEarg1 (containing argB, argC, argH and argE genes) as compared to the pEarg1-carrying strains.

Example 2

(1) Preparation of plasmid DNA containing argG gene of Escherichia coli:

Chromosomal DNA containing argG gene was isolated from Escherichia coli WA802 (FERM BP-718) described above in a conventional manner [Biochim. Biophys. Acta, 72, 619 (1963)].

pBR322 (ampicillin-resistant and tetracyclin-resistant) used as a vector was commercially available from Takara Shuzo Co., Ltd. Sixteen units of BanIII (product of Toyobo Co., Ltd.) were added to 100 $\mu\ell$ of a reaction solution for restriction enzyme BanIII (10 mM Tris, 50 mM NaCl and 7 mM MgCl$_2$, pH 7.5) containing 1 $\mu$g of pBR322 plasmid DNA and 3 $\mu$g of the chromosomal DNA as mentioned above. After incubation at 37°C for 60 minutes, the reaction mixture was heated at 65°C for 40 minutes to stop the reaction. To the digested reaction product were added 12 $\mu\ell$ of T4 ligase buffer solution at a 10-fold concentration, 3 $\mu\ell$ of 100 mM ATP and 350 units of T4 ligase (product of Takara Shuzo Co., Ltd.) followed by reaction at 12°C for 16 hours. This ligase reaction mixture was used for transformation of Escherichia coli EA-51 requiring arginine and methionine, obtained in Example 1, (1). Competent cells of the EA-51 strain were prepared by the method described in Example 1, (2). As a selection medium, Davis minimal agar medium supplemented with 30 $\mu$g/mℓ methionine and 50 $\mu$g/mℓ ampicillin was used. From the culture cells of the appearing transformants, plasmid DNA was isolated by the method of Ann, et al. described above. The plasmid obtained from the transformants and named pEarg3 was analyzed by digestion with various restriction enzymes and agarose gel electrophoresis. As the result, it was found that the plasmid has a structure wherein a BanIII DNA fragment of 7 kilobase is inserted at the single cleavage site with BanIII of pBR322 (the cleavage recognition site was the same as ClaI). The EA-51 strain was retransformed with pEarg3 in a manner as described above; all of the transformants selected by ampicillin resistance showed no arginine-requirement. From this fact, it was confirmed that argG gene isolated from Escherichia coli K12 was cloned. The steps for constructing pEarg3 are illustrated in Figure 1.

(2) Preparation of recombinant plasmid of pEarg2 and argG gene of Escherichia coli

pEarg2 prepared in Example 1, (2) and argG gene cloned in Example 2, (1) were recombined to prepare plasmid pEarg4 containing all of argB, argC, argH and argG genes.

To 100 $\mu\ell$ of a reaction solution for restriction enzyme BanIII containing 2 $\mu$g of pEarg2 DNA were added 0.3 units of BanIII. After reacting at 37°C for 60 minutes, the reaction mixture was heated at 65°C for 40 minutes to stop the reaction. On the other hand, 4 units of BanIII were added to 100 $\mu\ell$ of a reaction solution for restriction enzyme BanIII containing 2 $\mu$g of pEarg3 DNA. After reacting at 37°C for 60 minutes, the reaction mixture was heated at 65°C for 40 minutes to stop the reaction. After both digested reaction products had been mixed, 23 $\mu\ell$ of T4 ligase reaction buffer solution at a 10-fold concentration, 5 $\mu\ell$ of 100 mM ATP and 350 units of T4 ligase were added to the mixture followed by reacting at 12°C for 16 hours. This ligase reaction mixture was used for transformation of EA-51 strain. As a selection medium, Davis minimal agar medium supplemented with 30 $\mu$g/mℓ methionine and 25 $\mu$g/mℓ kanamycin was used. Plasmid obtained from the culture cells of the appearing transformants and named pEarg4 was analyzed by digestion with various restriction enzymes and agarose gel electrophoresis. As the result, it was found that the plasmid has a structure wherein a BanIII DNA fragment of 7 kilobase originating from pEarg3 is inserted at the BanIII-cleavage site outside the gene involved in kanamycin-resistance of pEarg2 having the two BanIII-cleavage sites. EA-21, EA-35, EA-77 and EA-51 obtained in Example 1, (1) were transformed with pEarg4; all of the transformants selected by kanamycin resistance showed no arginine-requirement. From

9

this fact, it was recognized that pEarg4 contains argB, argC, argH and argG genes.

Further with pEarg4, Corynebacterium glutamicum LA-A36 (requiring arginine) was transformed. The strain is arginine-requiring strain derived from lysozyme-sensitive mutant Corynebacterium glutamicum ATCC 31834 derived from ATCC 31833 by a conventional mutation treatment. It is assumed that the mutation in LA-A36 results from deficiency of ASS (corresponding to the argG gene product of Escherichia coli K12), because citrulline as an intermediate for arginine biosynthesis cannot be changed to arginine as a substance required for growth and because arginine-requirement is not complemented notwithstanding introduction of pEarg2. The seed culture, 0.1 mℓ, of Corynebacterium glutamicum LA-A36 was inoculated in 10 mℓ of NB medium. When OD reached 0.6, the cells were collected and the same procedure as in Example 1, (3) was carried out to transform LA-A36 strain with pEarg4. As a selection medium, RCGP agar medium containing 400 μg/mℓ kanamycin was employed. With respect to the thus obtained kanamycin-resistant transformants arginine-requirement was examined. As the result, all of the transformants showed no arginine-requirement. Accordingly, it was recognized that the argG gene was also expressed in the bacteria belonging to the genus Corynebacterium or Brevibacterium. The steps for constructing pEarg4 are illustrated in Figure 1.

(3) Production of L-arginine by pEarg4-carrying strain:

Protoplasts of Corynebacterium glutamicum ATCC 31833, Corynebacterium herculis ATCC 13868 and Brevibacterium flavum ATCC 14067 were prepared in a manner similar to Example 1, (3) and pEarg4 was introduced therein.

Plasmid was isolated from these transformants in a manner similar to Example 1, (3) and analyzed by digestion with various restriction enzymes and agarose gel electrophoresis. As the result, it was found that the plasmid has the same structure of pEarg4. Corynebacterium glutamicum ATCC 31833/pEarg4 has been deposited as Corynebacterium glutamicum K63 (FERM BP-1113) in Fermentation Research Institute, Agency of Industrial Science and Technology, Japan.

Production test of L-arginine with pEarg4-carrying strains, pEarg2-carrying strains and strains carrying no plasmid was carried out in a manner similar to Example 1, (3).

The results are shown in Table 4.

<u>Table 4</u>

| Strain | Amount of L-Arginine Produced (mg/ml) |
|---|---|
| <u>Corynebacterium glutamicum</u> ATCC 31833 | 0 |
| <u>Corynebacterium glutamicum</u> ATCC 31833/pEarg2 (K62 FERM BP-1112) | 1.9 |
| <u>Corynebacterium glutamicum</u> ATCC 31833/pEarg4 (K63 FERM BP-1113) | 4.2 |
| <u>Corynebacterium herculis</u> ATCC 13868 | 0 |
| <u>Corynebacterium herculis</u> ATCC 13868/pEarg2 | 2.1 |
| <u>Corynebacterium herculis</u> ATCC 13868/pEarg4 | 4.5 |
| <u>Brevibacterium flavum</u> ATCC 14067 | 0 |
| <u>Brevibacterium flavum</u> ATCC 14067/pEarg2 | 1.4 |
| <u>Brevibacterium flavum</u> ATCC 14067/pEarg4 | 4.3 |

From the foregoing results, it is clearly noted that the productivity of L-arginine can be improved with the strains carrying plasmid pEarg4 obtained by recombination of pEarg2 (containing argB, argC and argH genes) and the DNA fragment containing the argG gene as compared to the pEarg2-carrying strains.

**Claims**

1. A process for producing an amino acid selected from the group consisting of L-arginine, L-citrulline and L-ornithine which comprises culturing a microorganism belonging to the genus Corynebacterium or Brevibacterium carrying a recombinant DNA consisting of the genes coding for N-acetylglutamate kinase, N-acetyl-$\gamma$-glutamylphosphate reductase and argininosuccinase of a microorganism belonging to the genus Escherichia and a vector DNA in a medium until recoverable amounts of said amino acid are accumulated in the culture broth and recovering said amino acid therefrom.

2. The process according to claim 1, wherein the amino acid is L-arginine and the recombinant DNA further consists of the gene coding for argininosuccinate synthetase of a microorganism belonging to the genus Escherichia.

3. A recombinant DNA consisting of the genes coding for N-acetylglutamate kinase, N-acetyl-$\gamma$-glutamyl-phosphate reductase and argininosuccinase of a microorganism belonging to the genus Escherichia

and a vector DNA.

4. The recombinant DNA according to claim 3, wherein said recombinant DNA is pEarg2 carried by Corynebacterium glutamicum K62 (FERM BP-1112).

5. A recombinant DNA consisting of the genes coding for N-acetylglutamate kinase, N-acetyl-$\gamma$-glutamyl-phosphate reductase, argininosuccinase and argininosuccinate synthetase of a microorganism belonging to the genus Escherichia and a vector DNA.

6. The recombinant DNA according to claim 5, wherein said recombinant DNA is pEarg4 carried by Corynebacterium glutamicum K63 (FERM BP-1113).

7. A microorganism belonging to the genus Corynebacterium or Brevibacterium carrying a recombinant DNA consisting of the genes coding for N-acetylglutamate kinase, N-acetyl-$\gamma$-glutamylphosphate reductase and argininosuccinase of a microorganism belonging to the genus Escherichia and a vector DNA.

8. A microorganism belonging to the genus Corynebacterium or Brevibacterium carrying a recombinant DNA consisting of the genes coding for N-acetylglutamate kinase, N-acetyl-$\gamma$-glutamylphosphate reductase, argininosuccinase and argininosuccinate synthetase of a microorganism belonging to the genus Escherichia and a vector DNA.

**Patentansprüche**

1. Verfahren zur Herstellung einer Aminosäure, ausgewählt aus der Gruppe bestehend aus L-Arginin, L-Citrullin und L-Ornithin, umfassend das Züchten eines zur Gattung Corynebacterium oder Brevibacterium gehörenden Mikroorganismus, der eine rekombinante DNA enthält, die aus den N-Acetylglutamatkinase, N-Acetyl-$\gamma$-glutamylphosphatreductase und Argininosuccinase codierenden Genen eines zur Gattung Escherichia gehörenden Mikroorganismus und einer Vektor-DNA besteht, in einem Medium bis gewinnbare Mengen der Aminosäure im Kulturmedium akkumuliert sind, und das Gewinnen der Aminosäure aus dem Kulturmedium.

2. Verfahren nach Anspruch 1, wobei die Aminosäure L-Arginin ist und die rekombinante DNA weiter aus dem die Argininosuccinatsynthetase codierenden Gen eines Mikroorganismus besteht, der zur Gattung Escherichia gehört.

3. Rekombinante DNA, bestehend aus N-Acetylglutamatkinase, N-Acetyl-$\gamma$-glutamylphosphatreduktase und Argininosuccinase codierenden Genen eines Mikroorganismus, der zur Gattung Escherichia gehört, und einer Vektor-DNA.

4. Rekombinante DNA nach Anspruch 3, wobei die rekombinante DNA pEarg2 von Corynebacterium glutamicum K62 (FERM BP-1112) ist.

5. Rekombinante DNA, bestehend aus den N-Acetylglutamatkinase, N-Acetyl-$\gamma$-glutamylphosphatredukta-se, Argininosuccinase und Argininosuccinatsynthetase codierenden Genen eines Mikroorganismus, der zur Gattung Escherichia gehört, und einer Vektor-DNA.

6. Rekombinante DNA nach Anspruch 5, wobei die rekombinante DNA pEarg4 des Corynebacterium glutamicum K63 (FERM BP-1113) ist.

7. Mikroorganismus, der zur Gattung Corynebacterium oder Brevibacterium gehört, der eine rekombinante DNA trägt, die aus den N-Acetylglutamatkinase, N-Acetyl-$\gamma$-glutamylphosphatreduktase und Argininosuccinase codierenden Genen eines Mikroorganismus, der zur Gattung Escherichia gehört, und einer Vektor-DNA besteht.

8. Mikroorganismus, der zur Gattung Corynebacterium oder Brevibacterium gehört, der eine rekombinante DNA trägt, die aus den N-Acetylglutamatkinase, N-Acetyl-$\gamma$-glutamylphosphatreduktase, Argininosucci-nase und Argininosuccinatsynthetase codierenden Genen eines Mikroorganismus, der zur Gattung

EP 0 259 858 B1

Escherichia gehört, und einer Vektor-DNA besteht.

**Revendications**

1. Procédé de production d'un acide aminé choisi dans le groupe constitué par la L-arginine, la L-citrulline et la L-ornithine, qui comprend le fait de cultiver, dans un milieu, un microorganisme appartenant au genre Corynebacterium ou Brevibacterium et portant un ADN recombinant constitué des gènes codant la N-acétylglutamate kinase, la N-acétyl-$\gamma$-glutamylphosphate réductase et l'argininosuccinase d'un microorganisme appartenant au genre Escherichia et d'un ADN vecteur, jusqu'à ce que des quantités récupérables dudit acide aminé se soient accumulées dans le bouillon de culture, et la récupération dudit acide aminé à partir de celui-ci.

2. Procédé selon la revendication 1, dans lequel l'acide aminé est la L-arginine et l'ADN recombinant comporte en outre le gène codant l'argininosuccinate synthétase d'un microorganisme appartenant au genre Escherichia.

3. ADN recombinant constitué des gènes codant la N-acétylglutamate kinase, la N-acétyl-$\gamma$-glutamylphosphate réductase et l'argininosuccinase d'un microorganisme appartenant au genre Escherichia, et d'un ADN vecteur.

4. ADN recombinant de la revendication 3, ledit ADN recombinant étant pEarg2 porté par Corynebacterium glutamicum K62 (FERM BP-1112).

5. ADN recombinant constitué des gènes codant la N-acétylglutamate kinase, la N-acétyl-$\gamma$-glutamylphosphate réductase, l'argininosuccinase et l'argininosuccinate synthétase d'un microorganisme appartenant au genre Escherichia, et d'un ADN vecteur.

6. ADN recombinant de la revendication 5, ledit ADN recombinant étant pEarg4 porté par Corynebacterium glutamicum K63 (FERM BP-1113).

7. Microorganisme appartenant au genre Corynebacterium ou Brevibacterium et portant un ADN recombinant constitué des gènes codant la N-acétylglutamate kinase, la N-acétyl-$\gamma$-glutamylphosphate réductase et l'argininosuccinase d'un microorganisme appartenant au genre Escherichia, et d'un ADN vecteur.

8. Microorganisme appartenant au genre Corynebacterium ou Brevibacterium et portant un ADN recombinant constitué des gènes codant la N-acétylglutamate kinase, la N-acétyl-$\gamma$-glutamylphosphate réductase, l'argininosuccinase et l'argininosuccinate synthétase d'un microorganisme appartenant au genre Escherichia, et d'un ADN vecteur.

13

FIG.1